# EUROPEAN PATENT SPECIFICATION

(11) **EP 1 216 754 B1**
(45) Date of publication and mention of the grant of the patent: **17.11.2004**
(21) Application number: 02002408.9
(22) Date of filing: 01.05.1998
(51) Int. Cl.: B01L 9/06, B01L 3/02, G01N 35/02, B01L 3/00

(54) **Reaction receptacle apparatus**
Reaktion Behälter Apparat
Appareil de reaction comprenant plusieurs recipients

(30) Priority: 02.05.1997 US 46800
(43) Date of publication of application: 26.06.2002
(62) Divisional of application: 98919968.2
(73) Proprietor: Gen-Probe Incorporated, San Diego, CA 92121-4362 (US)
(72) Inventor: Horner, Glenn A., Boulder, Colorado 80304 (US); Schneider, Robert E., Erie, CO 80516 (US); Tseo, Gus, San Diego, California 92130 (US); Johnson, Shirley J., Del Mar, California 92014 (US); Smith, Robert J., Louisville, Colorado 80027 (US)
(74) Representative: Viering, Jentschura & Partner

(56) References cited:
- US-A- 3 676 076
- US-A- 4 287 155
- US-A- 4 554 839
- US-A- 4 824 641

## Description

### FIELD OF THE INVENTION

The present invention relates to reaction receptacles useful for containing chemical or biological substances.

### BACKGROUND OF THE INVENTION

Reaction receptacles or test tubes are commonly used in the chemical and biological arts to perform a variety of types of assays in a contained space. Assays that commonly have one or more steps performed in reaction receptacles include chemical reactions, immunoassays, and nucleic acid-based assays. Examples of such reactions and assays are thoroughly described in the available literature and are well known to those skilled in the art. While reaction receptacles are generally manufactured and sold as individual units or test tubes, it is common for practitioners to use holding racks to conveniently and collectively organize a group of reaction receptacles for performing multiple assays simultaneously or sequentially. In some instances, multiple reaction receptacles are assembled as a unitary piece.

With most assays, a substance transfer device is used to dispense solutions into or remove solutions from reaction receptacles. The most familiar substance transfer devices are pipettes and aspirators including one or more tubular elements through which fluids are dispensed or withdrawn. When substance transfer devices are used in conducting a group of independent assays at about the same time or in close proximity to one another, there is always the concern that a substance transfer device will inadvertently serve as a vehicle in transferring substances or contaminants between reaction receptacles. An additional concern is that the practitioner will improperly add substances into or remove substances from a reaction receptacle. To minimize the risk of cross-contamination and pipetting and aspirating errors, practitioners must carefully monitor substance transfers and exercise nearly flawless precision when pipetting substances into or aspirating substances from reaction receptacles. Avoiding cross-contamination and pipetting and aspirating mistakes is particularly important when the assay is diagnostic in nature or is designed to provide information concerning the progress of a patient's disease over time or the success of a treatment regimen.

One way to limit opportunities for cross-contamination is to reduce the amount of surface area on the substance transfer device that can come into contact with the contents of a reaction receptacle. This objective can be achieved by using a contact-limiting element, such as a pipette tip, which essentially serves as a barrier between the outer surface of the pipette and the contents of a reaction receptacle. And by selecting a pipette tip of sufficient length and volume, contact between the pipette and contents of a reaction receptacle can be substantially eliminated. This is because substances from the reaction receptacle will be drawn into a portion of the pipette tip which falls below the bottom surface of the pipette. Of course, in most instances, it will also be important to have a single pipette tip dedicated to each reaction receptacle.

Where a number of pipette tips are used to perform multiple assays simultaneously or sequentially, practitioners typically need to position a supply of pipette tips at a location that can be conveniently accessed by at least one pipette. Providing a sufficient quantity of pipette tips becomes more complicated when the substance transfer device functions robotically in an automated (or partially automated) assay instrument. In an automated format, a large reserve of pipette tips may need to be placed in the instrument at a site that is accessible by the pipette, but which limits the total amount of space required. Accordingly, there is a need for pipette tips that are readily accessible by a robotic pipette without requiring the pipette to engage in complicated movements or to travel over substantial distances.

Another problem presented by conventional reaction receptacles is that they come packaged as individual test tubes that are not amenable to manipulation by an automated assay instrument. Individual reaction receptacles hinder throughput efficiency since the practitioner and instrument must each handle the reaction receptacles separately. And because conventional reaction receptacles are not provided with any structure that permits them to be manipulated by an automated instrument, reaction receptacles are generally stationed at one situs within the instrument and are not afforded any automated mobility. This lack of movement imposes certain architectural limitations and assay inefficiencies since the instrument must be designed around the positioning of the reaction receptacles. Accordingly, there is a need for a reaction receptacle apparatus which can be manipulated by an automated assay instrument, where the apparatus may include one reaction receptacle or plurality of reaction receptacles coupled together as a single operative unit.

### SUMMARY OF THE INVENTION

It is an object of the present invention to provide a reaction receptacle apparatus that meets one or more of the needs set forth above. This object is met by a reaction receptacle apparatus according to claim 1. The apparatus can be used to perform chemical or biological assays and comprises a plurality of receptacles for containing substances used in performing such assays. The reaction receptacles are operatively coupled to one another, either directly or indirectly, and are capable of interacting with a substance transfer device that dispenses substances into or withdraws substances from some or all of the plurality of reaction receptacles making up the reaction receptacle apparatus.

So that the substance transfer device can safely and efficiently dispense substances into or withdraw substances from the reaction receptacles, contact-limiting elements are provided. The contact-limiting elements are constructed and arranged to be operatively engaged by the substance transfer device to limit potentially contaminating contact between at least a portion of the substance transfer device and a potentially contaminating substance that is dispensed into or withdrawn from a reaction receptacle by the substance transfer device. One or more contact-limiting elements are associated with each of one or more of the reaction receptacles of the reaction receptacle apparatus.

The reaction receptacle apparatus is outfitted with one or more contact-limiting element holding structures, each contact-limiting element holding structure being preferably associated with a different contact-limiting element. Each of the contact-limiting element holding structures is constructed and arranged to (i) receive and removably hold the associated contact-limiting element in an operative orientation in proximity to the associated receptacle so as to be operatively engageable by the substance transfer device, and (ii) allow the associated contact-limiting element to be removed from the associated contact-limiting element holding structure when the associated contact-limiting element is operatively engaged by the substance transfer device.

Because the reaction receptacle apparatus is supplied with its own contact-limiting elements, an automated assay instrument can be constructed so that the substance transfer device avoids complex motions and conveniently engages the contact-limiting elements when the reaction receptacle apparatus is brought into an operative position within the instrument. An additional benefit is that the instrument does not have to be configured to receive a store of contact-limiting elements, and practitioners are spared having to monitor the volume of contact-limiting elements in an instrument while assays are being run.

Other features and characteristics of the present invention, as well as the methods of operation, functions of related elements of structure and the combination of parts, and economies of manufacture, will become more apparent upon consideration of the following description and the appended claims with reference to the accompanying drawings, all of which form a part of this specification, wherein like reference numerals designate corresponding parts in the various figures.

### BRIEF DESCRIPTION OF THE DRAWINGS

FIGURE 1 is a perspective view of a first embodiment of a reaction receptacle apparatus and contact-limiting element in the form of a tiplet embodying aspects of the present invention;
FIGURE 2 is a side elevation of a contact-limiting tiplet;
FIGURE 3 is a partial bottom view of the reaction receptacle apparatus of FIGURE 1 taken in the direction indicated by arrow "III" in FIGURE 1;
FIGURE 4 is a side elevation of a first alternate embodiment of the reaction receptacle apparatus of the present invention;
FIGURE 5 is a top view of the reaction receptacle apparatus of FIGURE 4;
FIGURE 6 is a cross-section in the direction "VI-VI" of FIGURE 4;
FIGURE 7 is a cross-section in the direction "VII-VII" in FIGURE 4;
FIGURE 8 is a cross-section in the direction "VIII-VIII" in FIGURE 4;
FIGURE 9 is a perspective view of an exemplary reaction receptacle apparatus manipulating device for manipulating a reaction receptacle apparatus according to the present invention;
FIGURE 10 is a side elevation, partially in cross-section, of the manipulating device of FIGURE 9 with a reaction receptacle apparatus resident therein;
FIGURE 11 is a side elevation, partially in cross-section, of an exemplary reaction receptacle apparatus processing device for processing a reaction receptacle apparatus according to the present invention;
FIGURE 12 is a partial side view of a reaction receptacle apparatus according to the present invention and a skewed wobbler plate for imparting an oscillatory vibration to the apparatus;
FIGURE 13 is a cross-section showing a reaction receptacle apparatus according to the present invention carried by a receptacle carrier structure within a receptacle apparatus processing device with a tubular element of the processing device engaging a contact-limiting tiplet disposed within a contact-limiting holding structure of the apparatus of the present invention;
FIGURE 14 is a cross-section showing the reaction receptacle apparatus disposed within the receptacle carrier structure with the tubular elements and the contact-limiting tiplet disposed on the end of the' tubular element inserted into the apparatus;

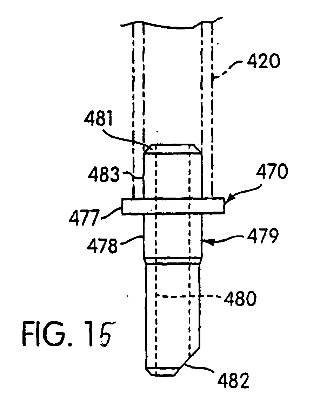

FIGURE 15 is a side elevation of an alternate embodiment of a contact-limiting tiplet engaged by a tubular element of a substance transfer device.

In the Figures referred to below, the reference numbers are not to be construed as limiting the claims.

### DETAILED DESCRIPTION OF THE PREFERRED EMBODIMENT

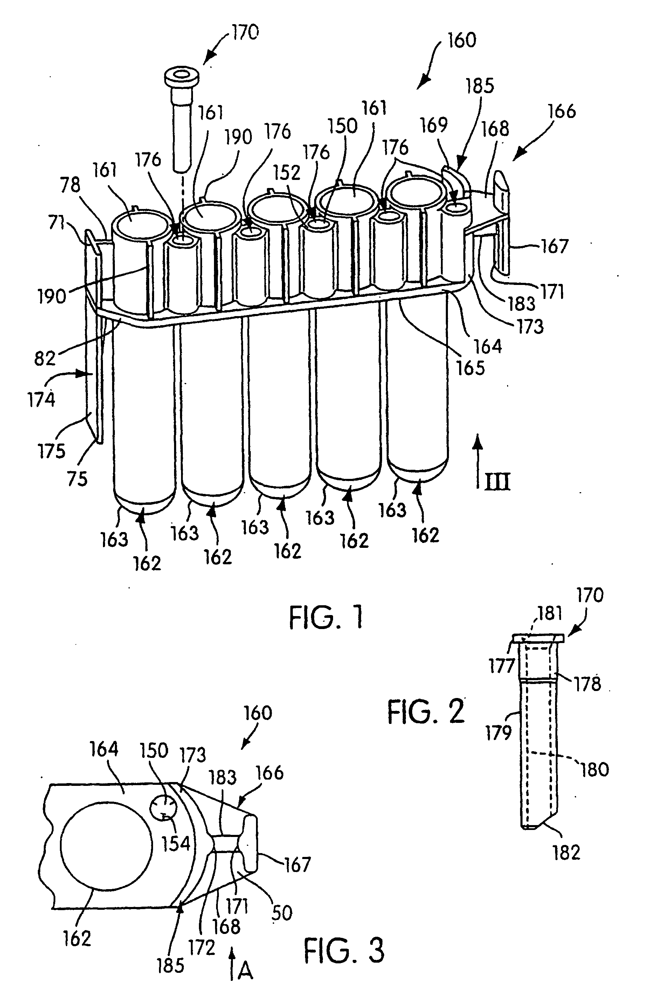

As shown in FIGURES 1 and 3, a preferred embodiment of a reaction receptacle apparatus according to the present invention is designated generally by the reference character 160. As shown, the reaction receptacle apparatus 160 comprises a plurality of individual receptacles 162. In the illustrated embodiment, the reaction receptacle apparatus 160 includes five individual receptacles 162, but a reaction receptacle according to the present invention may include any number of receptacles 162, as desired. Ten receptacles 162 are preferred and five receptacles 162 are most preferred. Each individual receptacle 162 preferably has a construction similar to that of a conventional test-tube, i.e., a cylindrical body with a circular open mouth 161 and a rounded closed bottom end 163. Each individual receptacle can, however, have other shapes, such as rectangular, octagonal. The receptacles may have the same or different shapes and sizes. The receptacles 162 are preferably oriented in an aligned arrangement comprising a single row of receptacles 162 and are connected to one another by a connecting rib structure 164 which defines a downwardly facing shoulder 165 extending longitudinally along either side of the reaction receptacle apparatus 160. The receptacles 162 may be oriented in a different nonlinear arrangement, or a single reaction receptacle apparatus may comprise more than one row of receptacles 162.

Reaction receptacle apparatus 160 is a single, integral piece formed of injection molded polypropylene. The most preferred polypropylene is sold by Montell Polyolefins, of Wilmington, Delaware, product number PD701NW. The Montell material is used because it is readily moldable and is chemically compatible with the preferred biological assays performed in the reaction receptacle apparatus. Moreover, the Montell material experiences a limited number of static discharge events, which is important when the results of the assay performed in the reaction receptacle apparatus are determined by the detection of light emitted by the contents of the apparatus at the conclusion of the assay. Static discharge events can interfere with accurate detection or quantification of the light output.

An arcuate shield structure 185 provided at one end of the reaction receptacle apparatus 160 includes an upper portion 169 and a lower portion 173. A receptacle apparatus manipulating structure 166, adapted to be engaged by a reaction receptacle manipulating device, extends from the shield upper portion 169. Receptacle apparatus manipulating structure 166 comprises a laterally extending plate 168 extending from shield upper portion 169 with a transverse piece 167 on the opposite end of the plate 168. A gusset wall 183 extends downwardly from lateral plate 168 between shield lower portion 173 and transverse piece 167.

As shown in FIGURE 3, the shield lower portion 173 and transverse piece 167 have mutually facing convex surfaces. The reaction receptacle apparatus 160 is preferably engaged by manipulating devices and other components, as will be described below, by moving an engaging member of the manipulating device laterally (in the direction "A") into a space 50 between the shield lower portion 173 and the transverse piece 167. The convex surfaces of the shield lower portion 173 and transverse piece 167 provide for wider points of entry for an engaging member undergoing a lateral relative motion into the space 50. Vertically extending, raised arcuate ridges 171, 172 may be provided in the middle of the convex surfaces of the transverse piece 167 and shield lower portion 173, respectively. The purpose of ridges 171, 172 will be described below.

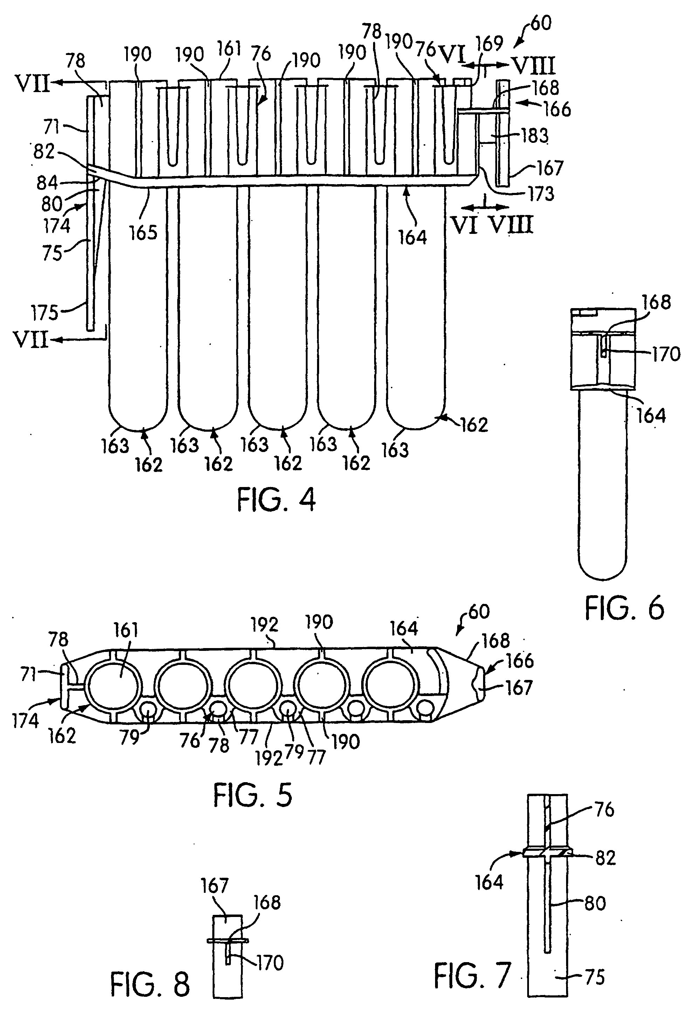

A label-receiving structure 174 provided on an end of the reaction receptacle apparatus 160 opposite receptacle apparatus manipulating structure 166 preferably includes an upper portion 71 and a lower portion 75, which together present a flat label-receiving surface 175. The label-receiving structure 174 further includes a vertical gusset wall 78 extending between upper portion 71 and the endmost receptacle 162 to provide a brace for the upper portion 71. As best shown in FIGURE 4, a gusset wall 80 of the label-receiving structure 174 is oriented vertically and extends diagonally from a location proximate rib structure 164 toward a lower end of lower portion 75 to provide a brace for lower portion 75. Labels, such as machine-scannable bar codes, can be applied to the surface 175 to provide identifying and instructional information on the reaction receptacle apparatus 160. Labels can be applied to surface 175 by any suitable means, such as, printing them onto surface 175 or adhering a label sheet, by means of an adhesive, to surface 175.

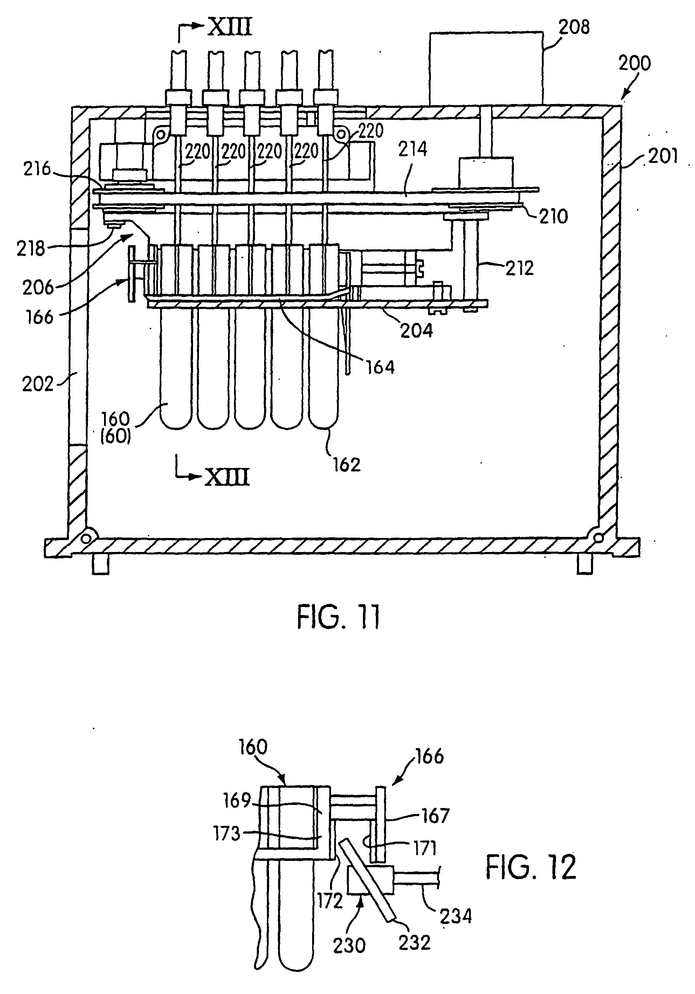

Substances can be dispensed into or removed from the receptacles 162 through their open mouths 161 by means of a substance transfer device, such as a pipetting or aspirating apparatus (hereinafter referred, to collectively as "pipetting apparatus or "pipette"). The pipetting apparatus may include a slender tubular element (see, e.g., tubular element 220 in FIGURE 11) that is inserted into the receptacle 162 through the open mouth 161 and which may come into contact with the receptacle 162 itself, the substance contained in the receptacle 162, and/or the substance being dispensed into the receptacle. A pipetting apparatus may be used to dispense substances into and/or remove substances from multiple individual receptacles 162. Accordingly, to reduce the likelihood of cross-contamination between individual receptacles 162, it is desirable to limit the amount of the pipetting apparatus that comes into contact with the substance or walls of any receptacle 162. Therefore, a contact-limiting element, which may take the form of a protective disposable tip, or tiplet, covers the end of the tubular element of the pipetting apparatus. One contact-limiting element is used to cover the end of the tubular element while the pipetting apparatus engages one individual receptacle to dispense substance into or withdraw substance from the receptacle. Before the pipetting apparatus moves to the next receptacle, that contact-limiting element is discarded or stored for later use with that receptacle, and a new contact-limiting element is engaged by the tubular element.

As shown in FIGURE 2, a preferred embodiment of a contact-limiting element comprises a tiplet 170. In the preferred embodiment, tiplet 170 comprises a tubular body 179 having a peripheral flange 177, preferably extending radially with respect to said tubular body 179, and a thickened wall portion 178, adjacent the peripheral flange 177, having a generally larger diameter than a remaining portion of the tubular body 179 of the tiplet 170. An axially extending inner bore 180 passes through the tiplet 170. Bore 180 includes an outwardly flared end 181, which facilitates insertion of a bottom free end of a tubular element of a pipetting apparatus into the bore 180 of tiplet 170. The inner diameter of inner bore 180 provides an interference fit with the outer diameter of the tubular element to frictionally secure tiplet 170 onto the tubular element when the bottom end of the tubular element is forced into the inner bore 180.

In the illustrated embodiment, the tubular body 179 and inner bore 180 are generally cylindrical in shape, consistent with the typically cylindrical shape of the tubular element of a substance transfer device, such as a pipetting or aspirating device. The present invention is not limited, however, to contact-limiting elements having tubular bodies and inner bores that are cylindrical, as the tubular body and inner bore of the contact-limiting element may have a shape that is other than cylindrical to conform to non-cylindrical tubular elements of substance transfer devices.

The bottom end of the tiplet 170 preferably includes a beveled portion 182. When tiplet 170 is used on the bottom of the tubular element of a pipetting apparatus used for aspirating substances from a receptacle 162, the beveled portion 182 will prevent a vacuum from forming between the end of the tiplet 170 and the bottom 163 of the receptacle 162.

An alternate embodiment of a contact-limiting element is a tiplet designated by reference number 470 in FIGURE 17. Tiplet 470 comprises a tubular body 479 having a peripheral flange 477, preferably extending radially with respect to said tubular body 479, and a thickened wall portion 478, adjacent the peripheral flange 477, of generally larger diameter than a remaining portion of the tubular body 479 of the tiplet 470. An axially extending inner bore 480 passes through the tiplet 470. Bore 480 includes a bevelled end 481, which facilitates insertion of an upper end 483 of the tubular body 479 into a bottom free end of a tubular element 420. The outer diameter of upper end 483 of the tubular body 479 provides an interference fit with the inner diameter of the tubular element 420 to frictionally secure tiplet 470 onto the tubular element 420 when the upper end 483 of the tubular body 479 is inserted into the bottom free end of the tubular element 420.

Again, the tubular body 479 and inner bore 480 need not necessarily be generally cylindrical in shape, as illustrated in FIGURE 17, may have a shape that is other than cylindrical to conform to non-cylindrical tubular elements of substance transfer devices.

The bottom end of the tiplet 470 preferably includes a beveled portion 482. When tiplet 470 is used on the bottom of the tubular element of a pipetting apparatus used for aspirating substances from a receptacle 162, the beveled portion 482 will prevent a vacuum from forming between the end of the tiplet 470 and the bottom 163 of the receptacle 162.

As shown in FIGURE 1, the reaction receptacle apparatus 160 preferably includes contact-limiting element holding structures in the form of tiplet holding structures 176 adjacent the open mouth 161 of each respective receptacle 162. Each tiplet holding structure 176 provides an elongated orifice 150, preferably generally cylindrical in shape, within which is received a contact-limiting tiplet 170 (470). An annular end face 152 extends about the orifice 150, and when the tiplet 170 (470) is inserted into a tiplet holding structure 176, the peripheral flange 177 (477) contacts the end face 152 of tiplet holding structure 176 to limit the depth to which the tiplet 170 (470) can be inserted into the orifice 150. The outside diameter of the thickened wall portion 178 (478) is slightly larger than inside diameter of the orifice 150. Alternatively, and preferably, a plurality of small, raised ribs 154 (see FIGURE 3) extend longitudinally along the inner wall of the orifice 150 at different circumferentially-spaced positions. The crests of the raised ribs 154 define an inner diameter that is slightly smaller than the outer diameter of the thickened wall portion 178 (478). Accordingly, the tiplet holding structure 176 provides a sliding interference fit between the thickened wall portion 178 (478) and the inner diameter of the orifice 150 or between the thickened wall portion 178 (478) and the crests of the ribs 154. Thus, tiplet 170 (470) is held securely within the orifice 150 of the tiplet holding structure 176 so the tiplet 170 (470) is unlikely to dislodge from the tiplet holding structure 176, even if the reaction receptacle apparatus 160 is inverted. On the other hand, if the tiplet 170 (470) is frictionally engaged by the tubular element of a pipetting apparatus while the tiplet 170 (470) is held in the tiplet holding structure 176, the frictional hold between the tiplet 170 (470) and the tubular element is greater than the frictional hold between the tiplet 170 (470) and the tiplet holding structure 176. Thus, the tiplet 170 (470) should remain secured on the end of the tubular element when the tubular element is withdrawn in an axial direction from the orifice 150 of the tiplet holding structure 176.

Throughout the remainder of this specification, reference will be made only to tiplet 170 (the embodiment shown in FIGURE 2). Those skilled in the art, however, will appreciate that the following descriptions and illustrations can apply to tiplet 470 (the embodiment shown in FIGURE 17) as well.

An alternate tiplet holding structure 76 is shown in FIGURES 4 and 5. Reaction receptacle apparatus 60 includes a tiplet holding structure 76 that is different from the tiplet holding structure 176 of reaction receptacle apparatus 160 of FIGURE 1. In all other respects, however, reaction receptacle apparatus 60 is identical to reaction receptacle apparatus 160. Tiplet holding structure 76 includes a tiplet-receiving orifice 79 with an end face 77 surrounding orifice 79 and forming a partial annulus. A slot 78 extends longitudinally along a wall of the tiplet holding structure 76. Slot 78 allows the tiplet holding structure 76 to expand when a tiplet 170 is inserted into the tiplet holding structure 76, and the resiliency of the material of which the reaction receptacle apparatus 60 is formed provides a frictional fit between a tiplet 170 and the tiplet holding structure 76.

As shown in FIGURES 1, 4, and 5, connecting rib structure 164 extends along both sides of the reaction receptacle apparatus 160 and defines downwardly facing shoulders 165 with outer edges 192 along each side of the reaction receptacle apparatus 160 (60). The reaction receptacle apparatus 160 (60) is operatively supported within a diagnostic instrument or the like by means of the shoulders 165 resting on parallel, horizontal flanges spaced apart from one another by a distance slightly greater than the width of the individual receptacle 162, but less than the width of the rib structure 164 between edges 192. Such flanges may be defined by a slot extending from an edge of a reaction receptacle apparatus supporting plate. In an automated instrument for processing the contents of a reaction receptacle apparatus, the reaction receptacle apparatus may be inserted into and removed from a supporting structure by a reaction receptacle apparatus manipulating device.

At an end of the rib structure 164 opposite the receptacle apparatus manipulating structure 166, two upwardly angled portions 82 provide upwardly angled shoulders 84 on both sides of the reaction receptacle apparatus 160 (60). The upwardly angled shoulders 84 facilitate sliding of the reaction receptacle apparatus 160 (60) onto a supporting structure.

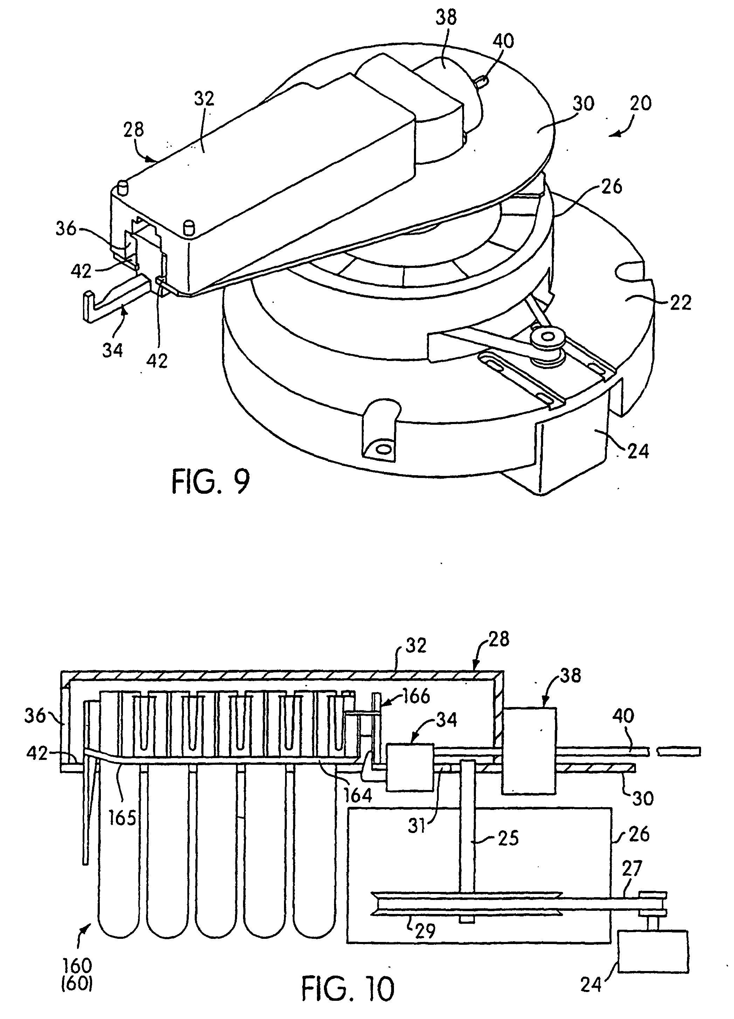

An exemplary device 20 for manipulating a reaction receptacle apparatus 160 (60) is shown in FIGURES 9 and 10. The device 20 includes a base structure 22 attached to a mounting bracket or mounting plate of an instrument which processes the contents of numerous reaction receptacle apparatuses according to the present invention and may perform one or more assays within each reaction receptacle apparatus 160. The manipulating device 20 moves the reaction receptacle apparatuses from one location to another within the instrument.

The manipulating device 20 further includes a rotating transport carrier 28 which rotates about a shaft 25 by means of a stepper motor 24 which turns a pulley 29 attached to the shaft 25 via a drive belt 27. The shaft 25 and pulley 29 may be covered by a pulley housing 26. The rotating transport carrier 28 includes a base plate 30 covered by a housing 32. The housing 32 includes an opening 36 at one end thereof, and the base plate 30 includes a slot 31 formed therein. A manipulating hook 34 is mounted for sliding translation in the slot 31 and is attached to a threaded drive screw 40 that is actuated by a stepper motor 38 to extend and retract the manipulating hook 34 within the slot 31.

To engage the receptacle apparatus manipulating structure 166 of the reaction receptacle apparatus 160, the manipulating hook 34 is extended to a forward position projecting from the opening 36 as shown in FIGURE 9. A lateral translation of the manipulating hook 34 is effected, such as by effecting a small rotation of the rotating transport carrier 28, to place the manipulating hook 34 in the space 50 between the lower portion 173 of the arcuate shield structure 185 and the transverse piece 167 of the receptacle apparatus manipulating structure 166. With the manipulating structure 166 engaged, the stepper motor 38 retracts the drive screw 40, pulling the manipulating hook 34 and the reaction receptacle apparatus 160 back into the rotating transport carrier 28. The downwardly facing shoulders 165 defined by the connecting rib structure 164 of the reaction receptacle apparatus 160 are supported by the base plate 30 along opposite edges 42 of the slot 31, thus supporting the reaction receptacle apparatus 160 in the rotating transport carrier 28. With the reaction receptacle apparatus 160 secured within the rotating transport carrier 28, the carrier 28 can be rotated by the stepper motor 24 to a different position at which the stepper motor 38 can extend the drive screw 40 and the manipulating hook 34 to push the reaction receptacle apparatus 160 out of the rotating transport carrier 28 and into a different location within the instrument.

An exemplary reaction receptacle processing device 200 is shown in FIGURE 11. Processing device 200 may represent one of many similar or related devices which together make up a reaction receptacle processing instrument.

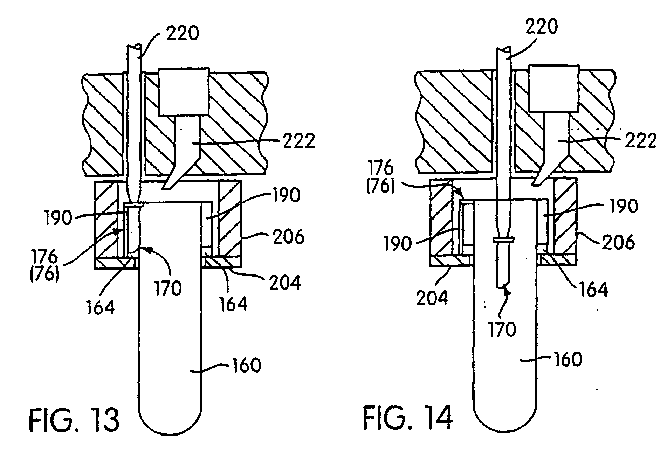

The processing device 200 includes a housing 201 with an opening 202 formed therein. A reaction receptacle apparatus 160 can be inserted into the processing device 200 through the opening 202 and removed through the opening 202 by a manipulating device such as the manipulating device 20 shown in FIGURES 9 and 10 and described above. Inside the housing 201 the reaction receptacle apparatus is supported by a receptacle carrier structure 206 having a base plate 204 (see also FIGURES 13 and 14) with a receptacle receiving slot (not shown) formed therein so that the reaction receptacle apparatus 160 can be supported by means of portions of the plate 204 along opposite edges of the slot supporting the connecting rib structure 164 of the reaction receptacle apparatus 160.

Processing device 200 may be a mixing. device for mixing the contents of the reaction receptacle apparatus 160; the processing device 200 may be a dispensing device for simultaneously dispensing substance into each of the individual receptacles 162 of the reaction receptacle apparatus 160; or the processing device 200 may be a device for simultaneously aspirating substance from each of the receptacles 162 of the reaction receptacle apparatus 160. Alternatively, the processing device 200 may perform any combination of two or more of the above functions.

As a mixing device, the receptacle carrier structure 206 may be coupled to an orbital mixing assembly comprising a stepper motor 208, a drive wheel 210 with an eccentric pin 212 extending therefrom, and an idler wheel 216 having an eccentric pin 218 and being coupled to the drive wheel 210 by means of a belt 214. As the stepper motor 208 rotates the drive pulley 210 which in turn rotates the idler pulley 216, the eccentric pins 212 and 218 engage the receptacle carrier structure 206 thus moving the receptacle carrier structure and the reaction receptacle apparatus 160 carried thereby in an orbital path of motion. Movement at a sufficiently high frequency can cause sufficient agitation of the reaction receptacle apparatus 160 to mix the contents thereof.

As shown in FIGURES 1, 4, and 5, lateral ribs 190 extend longitudinally along the outer walls of the receptacles 162 above the connecting rib structure 164 at diametrically opposed positions with respect to one another. The outer edges of the lateral ribs 190 are generally co-planar with the outer edges 192 of the connecting rib structure 164. The lateral ribs provide additional strength and rigidity to the open mouth 161 of the receptacle 162. In addition, the outer edges of the lateral ribs 190 can engage the sidewalls of a receptacle carrier structure 206, as shown in FIGURES 13 and 14, to limit the extent to which the reaction receptacle apparatus 160 will be allowed to tilt laterally within the receptacle carrier structure 206. Although it is generally preferred that lateral ribs 190 be provided on each of the receptacles 162, lateral ribs 190, when included, can be provided on less than all of the receptacles 162 as well.

When presented in the receptacle carrier structure 206, the reaction receptacle apparatus 160 can be engaged by a dispensing and/or aspirating system comprising an array of tubular elements 220. The dispensing and/or aspirating system preferably includes five tubular elements 220 oriented so as to correspond to the orientations of the individual receptacles 162 of the reaction receptacle apparatus 160. The tubular elements 220 are coupled to means for providing vertical movement of the free ends of the tubular elements 220 with respect to the reaction receptacle apparatus 160 to move the ends of the tubular elements 220 into and out of the individual receptacles 162 to aspirate and/or dispense substances. In addition, tubular elements 220 are coupled to means, such as a fluid pump and fluid source or a vacuum pump, for delivering fluid to each of the tubular elements 220 or providing a suction at each of the tubular elements 220.

As described above, however, before the tubular elements 220 are inserted into the reaction receptacles 162, it is preferred that a contact-limiting tiplet 170 be placed on the end of each tubular element 220. Accordingly, the tubular elements 220 are first lowered to simultaneously engage all of the tiplets 170 carried in their respective tiplet holding structures 176. The array of tubular elements 220 can be coupled to means for providing lateral translation of the tubular elements 220 for moving the tubular elements 220 to a position above the tiplet holding structures 176. Alternatively, the receptacle carrier structure 206 itself can be moved laterally to place the tiplet holding structures 176 below the respective tubular elements 220. Where the receptacle carrier structure 206 is coupled to an orbital mixing assembly as described above, the stepper motor 208 can move the assembly a limited number of steps, thus moving the receptacle carrier structure 206 and the reaction receptacle apparatus 160 a portion of one orbital path to place the tiplet holding structures 176 below the tubular elements 220 as shown in FIGURE 13.

As shown in FIGURES 1, 4, and 5, each of the respective tiplet holding structures 176 (76) is preferably disposed at a position between adjacent receptacles 162. Locating the tiplet holding structures 176 (76) between the adjacent receptacles 162 places the tubular elements 220 on the orbital paths of the contact-limiting element holding structures 176 (76) as the reaction receptacle apparatus 160 is moved with respect to the pipettes 220. Thus, the orbital mixer assembly can be used to properly position the tiplet holding structures 176 (76) with respect to the tubular elements 220, as described above. In addition, placing the tiplet holding structures 176 (76) between adjacent receptacles 162 provides for a narrower profile of the reaction receptacle apparatus 160 (60) than if the tiplet holding structures 176 (76) were located on the outer portion of the receptacles 162 nearest the edge 192 of the connecting rib structure 164.

The processing device 200 may also include an array of fixed nozzles 222 for dispensing substances into the receptacles 162 of the reaction receptacle apparatus 160 held in the receptacle carrier structure 206.

As shown in FIGURE 12, an alternate, oscillating mixing device 230 comprises a skewed wobbler plate 232 disposed on a shaft 234 driven by a motor (not shown). The reaction receptacle apparatus 160, carried by a carrier structure (not shown), is moved with respect to the oscillating mixing device 230 -- or the oscillating mixing 230 is moved with respect to the reaction receptacle apparatus 160 -- until the wobbler plate 232 is disposed in the space 50 between the lower portion 173 of the arcuate shield structure 185 and the transverse piece 167 of the receptacle apparatus manipulating structure 166. As the shaft 234 rotates, the position of the portion of the wobbler plate 232 engaged with the receptacle apparatus 160 varies in a linearly oscillating manner to impart a linear oscillating motion to the reaction receptacle apparatus 160.

The raised ridges 171, 172 provided in the middle of the convex surfaces of the transverse piece 167 and the lower portion 173, respectively, can minimize the surface contact between the wobbler plate 232 and the convex surfaces, thus limiting friction therebetween. It has been determined, however, that raised ridges 171, 172 can interfere with the engagement of the manipulating hook 34 of a manipulating device 20 with the apparatus manipulating structure 166. Therefore, raised ridges 171, 172 are preferably omitted.

While the invention has been described in connection with what is presently considered to be the most practical and preferred embodiments, it is to be understood that the invention is not to be limited to the disclosed embodiments.

## Claims

1. A receptacle apparatus (60, 160) for use in performing a chemical or biological assay, the apparatus (60, 160) comprising:
a plurality of aligned receptacles (162), each receptacle (162) comprising a hollow body, an open top end (161) and a closed bottom end (163), wherein the receptacles (162) are capable of interacting with a substance transfer device constructed and arranged to dispense fluid into or withdraw fluid from one or more of the receptacles (162);
one or more contact-limiting element holding structures (76, 176), each contact-limiting element holding structure (76, 176) being adjacent the open top end (161) of at least one of the receptacles (162) and comprising a body having an orifice (79, 150) therein for removably receiving a contact-limiting element (170, 470); and
a connecting rib structure (164) fixedly connecting the receptacles (162) to one another, wherein the receptacles (162), the contact-limiting element holding structures (76, 176) and the connecting rib structure (164) form a single, integral piece.

2. The receptacle apparatus (60, 160) of claim 1, wherein the receptacles (162) have different shapes and sizes.

3. The receptacle apparatus (60, 160) of claim 1, wherein the receptacles (162) have the same shape and size.

4. The receptacle apparatus (60, 160) of claim 1, wherein each receptacle (162) comprises a generally cylindrical body.

5. The receptacle apparatus (60, 160) of any one of claims 1 to 4, wherein the receptacles (162) are generally parallel to one another.

6. The receptacle apparatus (60, 160) of any one of claims 1 to 5, wherein the receptacles (162) are arranged to be simultaneously engageable by the substance transfer device to permit the substance transfer device to dispense fluid into or withdraw fluid from each of the receptacles (162) simultaneously.

7. The receptacle apparatus (60, 160) of any one of claims 1 to 6, wherein each contact-limiting element holding structure (76, 176) comprises a generally cylindrical body.

8. The receptacle apparatus (60, 160) of any one of claims 1 to 7, wherein the connecting rib structure (164) defines generally straight edge surfaces (192) extending along opposite sides of the aligned receptacles (162) and having generally flat shoulders (165) facing toward the bottom ends (163) of the receptacles (162).

9. The receptacle apparatus (60, 160) of claim 8, wherein one end of the connecting rib structure (164) is bent upwardly along opposite sides of the aligned receptacles (162) to define upwardly sloped edge surfaces (82) extending from the generally straight edge surfaces (192)

10. The receptacle apparatus (60, 160) of any one of claims 1 to 9 further comprising a label-receiving structure (174) having a label-receiving surface (175) for affixing human and/or machine readable information thereto.

11. The receptacle apparatus (60, 160) of any one of claims 1 to 10 further comprising a receptacle apparatus manipulating structure (166) which is constructed and arranged to be engaged by a receptacle apparatus manipulating device to manipulate the receptacle apparatus (60, 160).

12. The receptacle apparatus (60, 160) of any one of claims 1 to 11, wherein at least one of the receptacles (162) has ribs (190) extending outwardly from opposed outer surfaces thereof, the opposed surfaces corresponding to the opposite sides of the aligned receptacles (162).

13. The receptacle apparatus (60, 160) of any one of claims 1 to 12 further comprising one or more contact-limiting elements (170, 470), each contact-limiting element (170, 470) being associated with one of the contact-limiting element holding structures (76, 176), wherein the contact-limiting elements (170, 470) are constructed and arranged to be operatively engaged by the substance transfer device to limit contact between at least a portion of the substance transfer device and fluid dispensed into or withdrawn from one or more of the receptacles (162) by the substance transfer device.

14. The receptacle apparatus (60, 160) of claim 13, wherein each contact-limiting element (170, 470) comprises:
a tubular body (179, 479) having a proximal end, a distal end and an inner bore (180, 480) extending from the proximal end to the distal end; and
a peripheral flange (177, 477) disposed about an outer periphery of the tubular body (179, 479),
wherein each contact-limiting element (170, 470) is constructed and arranged to be frictionally secured onto a free end of a tubular element of the substance transfer device when the contact-limiting element (170, 470) is engaged by the tubular element.

15. The receptacle apparatus (60, 160) of claim 14, wherein a portion of the tubular body (179, 479) proximate the distal end is formed at an angle (182, 482) with respect to a longitudinal axis of the tubular body (179, 479).

16. The receptacle apparatus (60, 160) of claim 14 or 15, wherein:
the contact-limiting element holding structure (76, 176) has a peripheral end face (77, 152) oriented transversely to an axis of the orifice (79, 150) of the contact-limiting element holding structure (76, 176); and
the peripheral flange (177, 477) of the contact-limiting element (170, 470) is in contact with the peripheral end face (77, 152) of the contact-limiting element holding structure (76, 176) when the tubular body (179, 479) of the contact-limiting element (170, 470) is fully inserted into the orifice (79, 150) of the contact-limiting element holding structure (76, 176).

17. The receptacle apparatus (60, 160) of claim 13, wherein the contact-limiting element (170, 470) is a disposable protective tip.

18. The receptacle apparatus (60, 160) of claim 17, wherein the disposable protective tip is a pipette tip.

19. The receptacle apparatus (60, 160) of any one of claims 1 to 18 further comprising a lid structure constructed and arranged to enclose one or more of the receptacles (162).

## Patentansprüche

1. Eine Behältnisvorrichtung (60, 160) zur Verwendung bei der Durchführung von chemischen oder biologischen Tests, wobei die Vorrichtung aufweist:
eine Mehrzahl von ausgerichteten Behältnissen (162), wobei jedes Behältnis (162) einen Hohlkörper, ein offenes Kopfende (161) und ein geschlossenes Bodenende (163) umfasst, wobei die Behältnisse (162) mit einer Stoffüberführungsvorrichtung, die so ausgestaltet und angeordnet ist, um Flüssigkeit in ein oder mehrere der Behältnisse zu dispensieren oder aus diesen zu entnehmen, interagieren können;
eine oder mehrere Haltestrukturen für Kontakt-begrenzende Elemente (76, 176), wobei jede Haltestruktur für Kontakt begrenzende Elemente (76, 176) zum offenen Kopfende (161) von zumindest einem der Behältnisse (162) benachbart ist und einen Körper umfasst, der eine Öffnung (79, 150) zum wiederentfernbaren Aufnehmen eines Kontakt-begrenzenden Elements (170, 470) in sich aufweist; und
eine verbindende Rippenstruktur (164), welche die Behältnisse (162) fest miteinander verbindet, wobei die Behältnisse (162), die Haltestrukturen für Kontakt-begrenzende Elemente (76, 176) und die verbindende Rippenstruktur (164) ein einzelnes integrales Bauteil ausbilden.

2. Die Behältnisvorrichtung (60, 160) gemäß Anspruch 1, wobei die Behältnisse (162) unterschiedliche Formen und Größen aufweisen.

3. Die Behältnisvorrichtung (60, 160) gemäß Anspruch 1, wobei die Behältnisse (162) die gleiche Form und Größe aufweisen.

4. Die Behältnisvorrichtung (60, 160) gemäß Anspruch 1, wobei jedes Behältnis (162) einen im Allgemeinen zylindrischen Körper umfasst.

5. Die Behältnisvorrichtung (60, 160) gemäß einem der Ansprüche 1 bis 4, wobei die Behältnisse (162) im Allgemeinen parallel zueinander liegen.

6. Die Behältnisvorrichtung (60, 160) gemäß einem der Ansprüche 1 bis 5, wobei die Behältnisse (162) so angeordnet sind, dass sie mit der Stoffüberführungsvorrichtung gleichzeitig in Eingriff gebracht werden können, damit es der Stoffüberführungsvorrichtung ermöglicht wird, gleichzeitig Flüssigkeit in alle Behältnisse (162) zu dispensieren oder aus diesen zu entnehmen.

7. Die Behältnisvorrichtung (60, 160) gemäß einem der Ansprüche 1 bis 6, wobei jede Haltestruktur für Kontakt-begrenzende Elemente (76, 176) einen im Allgemeinen zylindrischen Körper umfasst.

8. Die Behältnisvorrichtung (60, 160) gemäß einem der Ansprüche 1 bis 7, wobei die verbindende Rippenstruktur (164) im Allgemeinen geradlinige Oberflächen (192) definiert, die sich auf gegenüberliegenden Seiten der ausgerichteten Behältnisse (162) erstrecken und im Allgemeinen breite Randleisten (165) aufweisen, die den Bodenenden (163) der Behältnisse (162) gegenüber liegen.

9. Die Behältnisvorrichtung (60, 160) gemäß Anspruch 8, wobei ein Ende der verbindenden Rippenstruktur (164) aufwärts entlang sich gegenüberliegender Seiten der ausgerichteten Behältnisse (162) gebogen ist, um aufwärts abgeschrägte Randoberflächen (82), die sich von den im Allgemeinen geradkantigen Oberflächen (192) weg erstrecken, zu definieren.

10. Die Behältnisvorrichtung (60, 160) gemäß einem der Ansprüche 1 bis 9, die ferner eine eine Markierung aufnehmende Struktur (174) mit einer eine Markierung aufnehmenden Oberfläche (175) zum Anbringen von für den Menschen und/oder Maschinen lesbaren Informationen umfasst.

11. Die Behältnisvorrichtung (60, 160) gemäß einem der Ansprüche 1 bis 10, die ferner eine Handhabungsstruktur für die Behältnisvorrichtung (166) umfasst, die so ausgestaltet und ausgerichtet ist, um mit einer Handhabungsvorrichtung für die Behältnisvorrichtung in Eingriff gebracht zu werden, um die Behältnisvorrichtung (60, 160) zu manipulieren.

12. Die Behältnisvorrichtung (60, 160) gemäß einem der Ansprüche 1 bis 11, wobei mindestens einer der Behältnisse (162) Rippen aufweist (190), die sich nach außen von sich einander gegenüberliegenden äußeren Oberflächen davon erstrecken, wobei die sich gegenüberliegenden Oberflächen den sich einander gegenüberliegenden Seiten der ausgerichteten Behältnisse (162) entsprechen.

13. Die Behältnisvorrichtung (60, 160) gemäß einem der Ansprüche 1 bis 12, die ferner ein oder mehrere Kontakt-begrenzende Elemente (170, 470) umfasst, wobei jedes Kontakt-begrenzende Element (170, 470) mit einer der Haltestrukturen für Kontakt-begrenzende Elemente (76, 176) verbunden ist, wobei die Kontakt-begrenzenden Elemente (170, 470) so ausgestaltet und angeordnet sind, um mit der Stoffüberführungsvorrichtung operativ in Eingriff gebracht zu werden, um den Kontakt zwischen mindestens einem Teil der Stoffüberführungsvorrichtung und der Flüssigkeit, die durch die Stoffüberführungsvorrichtung in ein oder mehrere der Behältnisse (162) dispensiert wird oder aus diesen entnommen wird, zu beschränken.

14. Die Behältnisvorrichtung (60, 160) gemäß Anspruch 13, wobei jedes Kontakt-begrenzende Element (170, 470) umfasst:
einen röhrenförmigen Körper (179, 479) mit einem proximalen Ende, einem distalen Ende und einer inneren Bohrung (180, 480), die sich vom proximalen Ende bis zum distalen Ende erstreckt; und
einem äußeren Flansch (177, 477) der über einem äußeren Rand des röhrenförmigen Körpers (179, 479) angebracht ist,
wobei jedes Kontakt-begrenzende Element (170, 470) so ausgestaltet und angeordnet ist, um reibschlüssig auf einem freien Ende eines röhrenförmigen Elementes der Stoffüberführungsvorrichtung gesichert zu sein, wenn das Kontakt begrenzende Element (170, 470) durch das röhrenförmige Element ergriffen wird.

15. Die Behältnisvorrichtung (60, 160) gemäß Anspruch 14, wobei ein Teil des röhrenförmigen Körpers (179, 479) benachbart zu dem distalen Ende in einem Winkel (182, 482) in Bezug auf die Längsachsen des röhrenförmigen Körpers (179, 479) ausgebildet ist.

16. Die Behältnisvorrichtung (60, 160) gemäß Anspruch 14 oder 15, wobei:
die Haltestruktur für Kontakt-begrenzende Elemente (76, 176) eine äußere Endfläche (77, 152) aufweist, die schräg zu einer Achse der Öffnung (79, 150) der Haltestruktur für Kontakt-begrenzende Elemente (76, 176) ausgerichtet ist; und
der äußere Flansch (177, 477) des Kontakt-begrenzenden Elements (170, 470) in Kontakt mit der äußeren Endfläche (77, 152) der Haltestruktur für Kontakt-begrenzende Elemente (76, 176) steht, wenn der röhrenförmige Körper (179, 479) des Kontakt-begrenzenden Elements (170, 470) vollständig in die Öffnung (79, 150) der Haltestruktur für Kontakt-begrenzende Elemente (76, 176) eingesteckt ist.

17. Die Behältnisvorrichtung (60, 160) gemäß Anspruch 13, wobei das Kontakt-begrenzende Element (170, 470) eine wegwerfbare Schutzspitze ist.

18. Die Behältnisvorrichtung (60, 160) gemäß Anspruch 17, wobei die wegwerfbare Schutzspitze eine Pipettenspitze ist.

19. Die Behältnisvorrichtung (60, 160) gemäß einem der Ansprüche 1 bis 18, die ferner eine Klappenstruktur umfasst, die so ausgestaltet und angeordnet ist, um ein oder mehrere Behältnisse (162) zu umschließen.

## Revendications

1. Dispositif à réceptacles (60, 160) à utiliser lors de la réalisation d'essais chimiques ou biologiques, le dispositif (60, 160) comprenant:
une pluralité de réceptacles alignés (162), chacun des réceptacles (162) comprenant un corps creux, une extrémité supérieure ouverte (161) et une extrémité inférieure fermée (163), dans lequel les réceptacles (162) peuvent interagir avec un dispositif de transfert de substance construit et agencé pour délivrer un liquide dans ou prélever un liquide d'un ou de plusieurs réceptacles (162);
une ou plusieurs structures de support d'élément limitant le contact (76, 176), chacune des structures de support d'élément limitant le contact (76, 176) étant adjacente à l'extrémité supérieure ouverte (161) d'au moins un des réceptacles (162) et comprenant un corps présentant un orifice (79, 150) dans celui-ci afin de recevoir de manière amovible un élément limitant le contact (170, 470) ; et
une structure de liaison à nervure (164) reliant les réceptacles (162) les uns aux autres, dans lequel les réceptacles (162), les structures de support d'élément limitant le contact (76, 176) et les structures de liaison à nervure (164) forment une pièce intégrale unique.

2. Dispositif à réceptacles (60, 160) selon la revendication 1, dans lequel les réceptacles (162) présentent des formes et tailles différentes.

3. Dispositif à réceptacles (60, 160) selon la revendication 1, dans lequel les réceptacles (162) présentent la même forme et la même taille.

4. Dispositif à réceptacles (60, 160) selon la revendication 1, dans lequel chaque réceptacle (162) comprend un corps globalement cylindrique.

5. Dispositif à réceptacles (60, 160) selon l'une quelconque des revendications 1 à 4, dans lequel les réceptacles (162) sont globalement parallèles les uns aux autres.

6. Dispositif à réceptacles (60, 160) selon l'une quelconques des revendications 1 à 5, dans lequel les réceptacles (162) sont agencés pour être simultanément en prise avec le dispositif de transfert de liquide pour permettre au dispositif de transfert de liquide de délivrer du liquide dans ou de prélever du liquide de chacun des réceptacles (162) simultanément.

7. Dispositif à réceptacles (60, 160) selon l'une quelconque des revendications 1 à 6, dans lequel chaque structure de support d'élément limitant le contact (76, 176) comprend un corps globalement cylindrique.

8. Dispositif à réceptacles (60, 160) selon l'une quelconque des revendications 1 à 7, dans lequel la structure de liaison à nervure (164) définit une surface de bordure globalement linéaire (192) s'étendant le long des côtés opposés des réceptacles (162) alignés, les épaulements globalement plans (165) étant tournés vers les extrémités inférieures (163) des réceptacles (162).

9. Dispositif à réceptacles (60, 160) selon la revendication 8, dans lequel une extrémité de ladite structure de liaison à nervure (164) est pliée vers le haut le long des côtés opposés des réceptacles alignés (162) pour définir des surfaces de bordure inclinées vers le haut (82) s'étendant à partir des surfaces de bordure globalement droites (192).

10. Dispositif à réceptacles (60, 160) selon l'une quelconque des revendications 1 à 9, comprenant en outre une structure pour recevoir une étiquette (174) présentant une surface de réception d'étiquette (175) pour y fixer des informations lisibles par un humain et/ou une machine.

11. Dispositif à réceptacles (60, 160) selon l'une quelconque des revendications 1 à 10, comprenant en outre une structure de manipulation du dispositif à réceptacles (166) qui est construite et agencée pour être en prise avec un dispositif de manipulation du dispositif à réceptacles, afin de manipuler le dispositif à réceptacles (60, 160).

12. Dispositif à réceptacles (60, 160) selon l'une quelconque des revendications 1 à 11, dans lequel au moins un des réceptacles (162) présente des nervures (190) s'étendant vers l'extérieur des surfaces extérieures opposées de celui-ci, les surfaces opposées correspondant aux côtés opposés des réceptacles alignés (162).

13. Dispositif à réceptacles (60, 160) selon l'une quelconque des revendications 1 à 12 comprenant en outre un ou plusieurs éléments limitant le contact (170, 470), chacun des éléments limitant le contact (170, 470) étant associé à l'une des structures de support d'élément limitant le contact (76, 176), dan lesquel les éléments limitant le contact (170, 470) sont construits et agencés pour être mis en prise opérationnelle avec le dispositif de transfert de substance pour limiter le contact entre au moins une partie du dispositif de transfert de substance et le liquide délivré ou prélevé d'un ou de plusieurs récipients (162) par le dispositif de transfert de substance.

14. Dispositif à réceptacles (60, 160) selon la revendication 13, dans lequel chacun des éléments limitant le contact (170, 470) comprend:
un corps tubulaire (179, 479) présentant une extrémité proximale, une extrémité distale et un alésage intérieur (180, 480) s'étendant de l'extrémité proximale à l'extrémité distale ; et
une bride périphérique (177, 477) disposée autour d'une périphérie extérieure du corps tubulaire (179, 479),
dans lequel chaque élément limitant le contact (170, 470) est construit et agencé pour être fixé par friction sur une extrémité libre d'un élément tubulaire du dispositif de transfert de substance lorsque ledit élément limitant le contact (170, 470) est en prise avec l'élément tubulaire.

15. Dispositif à réceptacles (60, 160) selon la revendication 14, dans lequel une partie du corps tubulaire (179, 479) à proximité de l'extrémité distale est formée à un angle (182, 482) par rapport à un axe longitudinal du corps tubulaire (179,479).

16. Dispositif à réceptacles (60, 160) selon la revendication 14 ou 15, dans lequel :
la structure de support d'élément limitant le contact (76, 176) a une face d'extrémité périphérique (77, 152) orientée transversalement à un axe de l'orifice (79, 150) de la structure du support d'élément limitant le contact (76, 176) ; et
la bride périphérique (177, 477) de l'élément limitant le contact (170, 470) est en contact avec la face d'extrémité périphérique (77, 152) de la structure de support d'élément limitant le contact (76, 176) lorsque le corps tubulaire (179, 479) de l'élément limitant le contact (170, 470) est entièrement inséré dans l'orifice (79, 150) de la structure de maintien de l'élément limitant le contact (76, 176).

17. Dispositif à réceptacles (60, 160) selon la revendication 13, dans lequel l'élément limitant le contact (170, 470) est un embout de protection jetable.

18. Dispositif à réceptacles (60, 160) selon la revendication 17, dans lequel l'embout de protection jetable est un embout de pipette.

19. Dispositif à réceptacles (60, 160) selon l'une quelconque des revendications 1 à 18, comprenant en outre une structure de couvercle construite et agencée pour comprendre un ou plusieurs réceptacles (162).
